# EUROPEAN PATENT SPECIFICATION

(11) **EP 1 278 851 B1**
(45) Date of publication and mention of the grant of the patent: **18.01.2006**
(21) Application number: 01931876.5
(22) Date of filing: 21.05.2001
(51) Int. Cl.: C12N 15/13, C12N 15/62, C07K 16/28, C07K 16/46, C07K 19/00, C12N 15/85, C12N 5/10, A61K 39/395, A61P 35/00

(54) **HUMANISED ANTIBODIES TO THE EPIDERMAL GROWTH FACTOR RECEPTOR**
HUMANISIERTE ANTIKÖRPER GEGEN DEN EPIDERMALEN WACHSTUMSFAKTORREZEPTOR
ANTICORPS HUMANISES CONTRE LE RECEPTEUR DU FACTEUR DE CROISSANCE EPIDERMIQUE

(30) Priority: 19.05.2000 GB 0011981; 24.08.2000 GB 0020794
(43) Date of publication of application: 29.01.2003
(73) Proprietor: Scancell Limited, Nottingham NG5 1QA (GB)
(72) Inventor: ELLIS, John Robert Maxwell, Nottingham NG5 1QA (GB); DURRANT, Linda Gillian, Nottingham NG5 1QA (GB)
(74) Representative: Lee, Nicholas John
(86) International application number: PCT/GB2001/002226
(87) International publication number: WO 2001/088138

(56) References cited:
- EP-A- 0 519 596
- EP-A- 0 586 002
- EP-A- 0 699 755
- EP-A- 0 706 799
- EP-A- 0 712 863
- L. DURRANT ET AL.: "Screening of monoclonal antibodies recognizing oncofetal antigens for isolation of trophoblasts from maternal blood for prenatal diagnosis." PRENATAL DIAGNOSIS, vol. 14, 1994, pages 131-140, XP000197363 Chichester, GB cited in the application
- C. MATEO ET AL.: "Humanization of a mouse monoclonal antibody that blocks the epidermal growth factor receptor: recovery of antagonistic activity." IMMUNOTECHNOLOGY, vol. 3, no. 1, March 1997 (1997-03), pages 71-81, XP004075440 Amsterdam, The Netherlands
- G. MARK ET AL.: "CDR-grafting and veneering approaches to MAb humanization." ABSTRACT OF PAPERS. AMERICAN CHEMICAL SOCIETY, vol. 207, no. 1-2, 1994, XP001024025 Washington, DC, USA
- J. HAUNSCHILD ET AL.: "Pharmacokinetics of reshaped mAb 425 in three animal species." CELL BIOPHYSICS, vol. 26, no. 3, June 1995 (1995-06), pages 167-182, XP001024019 London, GB

### SEQUENCE LISTING

<110> Scancell Limited
   Ellis, John Robert Maxwell
   Durrant, Linda Gillian
<120> Humanised Antibodies
<130> P32555WO/NJL
<140> PCT/GB01/02226
   <141> 2001-05-21
<150> GB 0011981.8
   <151> 2000-05-19
<150> GB 0020794.4
   <151> 2000-08-24
<160> 28
<170> Patent In version 3.1
<210> 1
   <211> 363
   <212> DNA
   <213> Mus musculus
<400> 1
   caggtgcagc tgcaggagtc tgggggaggc ttagtgaagg ctggagggtc cctgaaactc 60
   tcctgtgcag cctctggatt cgctttcaat acctatgaca tgtcttgggt tcgccagact 120
   ccggagaaga ggctggagtg ggtcgcatac attggtagtg gtggtgatag aacctactat 180
   ccagacactg tgaagggccg attcaccatt tccagagaca atggcaagaa caccctgtat 240
   ttgcaattga acagtctgaa gtctgaggac acagccatgt attactgtgc aagacattat 300
   ggtcactacg tggactatgc tgtggactac tggggtcaag gaaccacggt caccgtctcc 360
   tca 363
<210> 2
   <211> 336
   <212> DNA
   <213> Mus musculus
<400> 2
   gatgtgttga tgacccaaac tccactctct ctgcctgtca gtcttgggga tcaagcctcc 60
   atctcttgca gatctagtca gagcattgta catagtaatg gaaacaccta tttagaatgg 120
   tacctgcaga aaccaggcca gtctccaaag ctcctgatct acaaagtttc caaccgattt 180
   tctggggtcc cagacagatt cagtggcagt ggatcaggga cagatttcac actcaagatc 240
   agcagagtgg aggctgagga tctgggaatt tattactgct ttcaaggttc acatgttccg 300
   tggacgttcg gtggaggcac caagctggaa atcaac 336
<210> 3
   <211> 121
   <212> PRT
   <213> Mus musculus
<400> 3
   Gln Val Gln Leu Gln Glu Ser Gly Gly Gly Leu Val Lys Ala Gly Gly
   1               5                   10                  15
   Ser Leu Lys Leu Ser Cys Ala Ala Ser Gly Phe Ala Phe Asn Thr Tyr
               20                  25                  30
   Asp Met Ser Trp Val Arg Gln Thr Pro Glu Lys Arg Leu Glu Trp Val
           35                  40                  45
   Ala Tyr Ile Gly Ser Gly Gly Asp Arg Thr Tyr Tyr Pro Asp Thr Val
       50                  55                  60
   Lys Gly Arg Phe Thr Ile Ser Arg Asp Asn Gly Lys Asn Thr Leu Tyr
   65                  70                  75                  80
   Leu Gln Leu Asn Ser Leu Lys Ser Glu Asp Thr Ala Met Tyr Tyr Cys
                   85                  90                  95
   Ala Arg His Tyr Gly His Tyr Val Asp Tyr Ala Val Asp Tyr Trp Gly
               100                 105                 110
   Gln Gly Thr Thr Val Thr Val Ser Ser
           115                 120
<210> 4
   <211> 112
   <212> PRT
   <213> Mus musculus
<400> 4
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                   60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Ile Tyr Tyr Cys Phe Gln Gly
                   85                  90                  95
   Ser His Val Pro Trp Thr Phe Gly Gly Gly Thr Lys Leu Glu Ile Asn
               100                 105                 110
<210> 5
   <211> 37
   <212> DNA
   <213> Artificial
<220>
   <221>
   <223> Primer
<400> 5
   gcatgttgac cctgacgcaa gcttatgaat atgcaaa 37
<210> 6
   <211> 27
   <212> DNA
   <213> Artificial
<220>
   <221>
   <223> Primer
<400> 6
   gactcctgca gctggacctg ggagtgg 27
<210> 7
   <211> 31
   <212> DNA
   <213> Artificial
<220>
   <221>
   <223> Primer
<400> 7
   ccaactccca ggtccagctg caggagtctg g 31
<210> 8
   <211> 23
   <212> DNA
   <213> Artificial
<220>
   <221>
   <223> Primer
<400> 8
   ctcacctgag gagacggtga ccg 23
<210> 9
   <211> 23
   <212> DNA
   <213> Artificial
<220>
   <221>
   <223> Primer
<400> 9
   cggtcaccgt ctcctcaggt gag 23
<210> 10
   <211> 36
   <212> DNA
   <213> Artificial
<220>
   <221>
   <223> Primer
<400> 10
   gcgatagctg gactgaatgg atcctataaa tctctg 36
<210> 11
   <211> 33
   <212> DNA
   <213> Artificial
<220>
   <221>
   <223> Primer
<400> 11
   gggtcatcaa cacatcggag tggacacctg tgg 33
<210> 12
   <211> 30
   <212> DNA
   <213> Artificial
<220>
   <221>
   <223> Primer
<400> 12
   caggtgtcca ctccgatgtg ttgatgaccc 30
<210> 13
   <211> 35
   <212> DNA
   <213> Artificial
<220>
   <221>
   <223> Primer
<400> 13
   gtttaaattc tactcacgtt tgatttccag cttgg 35
<210> 14
   <211> 35
   <212> DNA
   <213> Artificial
<220>
   <221>
   <223> Primer
<400> 14
   ccaagctgga aatcaaacgt gatgagaatt taaac 35
<210> 15
   <211> 36
   <212> DNA
   <213> Artificial
<220>
   <221>
   <223> Primer
<400> 15
   gcgatagctg gactgaatgg atccaactga ggaagc 36
<210> 16
   <211> 18
   <212> DNA
   <213> Artificial
<220>
   <221>
   <223> Primer
<400> 16
   gcatgttgac cctgacgc 18
<210> 17
   <211> 18
   <212> DNA
   <213> Artificial
<220>
   <221>
   <223> Primer
<400> 17
   gcgatagctg gactgaat 18
<210> 18
   <211> 120
   <212> PRT
   <213> Mus musculus
<400> 18
   Gln Val Gln Leu Val Glu Thr Gly Gly Gly Leu Ile Gln Pro Gly Gly
   1               5                   10                  15
   Ser Leu Pro Leu Ser Cys Ala Ala Ser Gly Phe Ala Phe Asn Thr Tyr
               20                  25                  30
   Asp Met Ser Trp Val Arg Gln Ala Pro Gly Lys Gly Leu Glu Trp Val
           35                  40                  45
   Ala Tyr Ile Gly Ser Gly Gly Asp Arg Thr Tyr Tyr Pro Asp Thr Val
       50                  55                  60
   Lys Gly Arg Phe Thr Ile Ser Arg Asp Asn Ser Lys Asn Thr Leu Tyr
   65                  70                  75                  80
   Leu Gln Leu Asn Ser Leu Arg Ala Glu Asp Thr Ala Val Tyr Tyr Cys
                   85                  90                  95
   Ala Arg His Tyr Gly Tyr Val Asp Tyr Ala Val Asp Tyr Trp Gly Gln
               100                 105                 110
   Gly Thr Thr Val Thr Val Ser Ser
           115                 120
<210> 19
   <211> 120
   <212> PRT
   <213> Mus musculus
<400> 19
   Gln Val Gln Leu Val Glu Thr Gly Gly Gly Leu Ile Gln Pro Gly Gly
   1 5 10 15
   Ser Leu Arg Met Ser Cys Ala Ala Ser Gly Phe Ala Phe Asn Thr Tyr
   20 25 30
   Asp Met Ser Trp Val Arg Gln Ala Pro Gly Lys Gly Leu Glu Trp Val
   35 40 45
   Ala Tyr Ile Gly Ser Gly Gly Asp Arg Thr Tyr Tyr Pro Asp Thr Val
   50 55 60
   Lys Gly Arg Phe Thr Ile Ser Arg Asp Asn Ser Lys Asn Thr Leu Tyr
   65 70 75 80
   Leu Gln Leu Asn Ser Leu Arg Ala Glu Asp Thr Ala Val Tyr Tyr Cys
   85 90 95
   Ala Arg His Tyr Gly Tyr Val Asp Tyr Ala Val Asp Tyr Trp Gly Gln
   100 105 110
   Gly Thr Thr Val Thr Val Ser Ser
   115 120
<210> 20
   <211> 120
   <212> PRT
   <213> Mus musculus
<400> 20
   Gln Val Gln Leu Val Glu Thr Gly Gly Gly Leu Ile Gln Pro Gly Gly
   1 5 10 15
   Ser Leu Arg Met Ser Cys Ala Ala Ser Gly Phe Ala Phe Asn Thr Tyr
   20 25 30
   Asp Met Ser Trp Val Arg Gln Ala Pro Gly Lys Gly Leu Glu Trp Ile
   35 40 45
   Ala Tyr Ile Gly Ser Gly Gly Asp Arg Thr Tyr Tyr Pro Asp Thr Val
   50 55 60
   Lys Gly Arg Phe Thr Ile Ser Arg Asp Asn Ser Lys Asn Thr Leu Tyr
   65 70 75 80
   Leu Gln Leu Asn Ser Leu Arg Ala Glu Asp Thr Ala Val Tyr Tyr Cys
   85 90 95
   Ala Arg His Tyr Gly Tyr Val Asp Tyr Ala Val Asp Tyr Trp Gly Gln
   100 105 110
   Gly Thr Thr Val Thr Val Ser Ser
   115 120
<210> 21
   <211> 120
   <212> PRT
   <213> Mus musculus
<400> 21
   Gln Val Gln Leu Val Glu Thr Gly Gly Gly Leu Ile Gln Pro Gly Gly
   1 5 10 15
   Ser Leu Arg Met Ser Cys Ala Ala Ser Gly Phe Ala Phe Asn Thr Tyr
   20 25 30
   Asp Met Ser Trp Val Arg Gln Ala Pro Gly Lys Gly Leu Glu Trp Ile
   35 40 45
   Ala Tyr Ile Gly Ser Gly Gly Asp Arg Thr Tyr Tyr Pro Asp Thr Val
   50 55 60
   Lys Gly Arg Phe Thr Ile Ser Arg Asp Asn Ser Lys Asn Thr Leu Tyr
   65 70 75 80
   Leu Gln Leu Asn Ser Leu Arg Ala Glu Asp Thr Ala Val Tyr Tyr Cys
   85 90 95
   Ala Arg His Tyr Gly Tyr Ala Asp Tyr Ala Val Asp Tyr Trp Gly Gln
   100 105 110
   Gly Thr Thr Val Thr Val Ser Ser
   115 120
<210> 22
   <211> 120
   <212> PRT
   <213> Mus musculus
<400> 22
   Gln Val Gln Leu Gln Glu Ser Gly Gly Gly Leu Val Lys Ala Gly Gly
   1 5 10 15
   Ser Leu Lys Leu Ser Cys Ala Ala Ser Gly Phe Ala Phe Asn Thr Tyr
   20 25 30
   Asp Met Ser Trp Val Arg Gln Thr Pro Glu Lys Arg Leu Glu Trp Val
           35                  40                  45
   Ala Tyr Ile Gly Ser Gly Gly Asp Arg Thr Tyr Tyr Pro Asp Thr Val
       50                  55                  60
   Lys Gly Arg Phe Thr Ile Ser Arg Asp Asn Gly Lys Asn Thr Leu Tyr
   65                  70                  75                  80
   Leu Gln Leu Asn Ser Leu Lys Ser Glu Asp Thr Ala Met Tyr Tyr Cys
                   85                  90                  95
   Ala Arg His Tyr Gly Tyr Val Asp Tyr Ala Val Asp Tyr Trp Gly Gln
               100                 105                 110
   Gly Thr Thr Val Thr Val Ser Ser
           115                 120
<210> 23
   <211> 53
   <212> PRT
   <213> Mus musculus
<400> 23
   Asn Ser Tyr Pro Gly Cys Ala Arg His Tyr Gly His Tyr Cys Leu Asn
   1               5                   10                  15
   Gly Gly Val Cys Met His Ile Gly Ser Leu Asp Ser Tyr Thr Cys Asn
               20                  25                  30
   Cys Val Val Asp Tyr Ala Val Asp Arg Cys Gln Thr Arg Asp Leu Arg
           35                  40                  45
   Tyr Trp Gly Leu Gln
       50
<210> 24
   <211> 53
   <212> PRT
   <213> Mus musculus
<400> 24
   Asn Ser Tyr Pro Gly Cys Pro Ser Ser Tyr Ala Gly Tyr Cys Leu Asn
   1 5 10 15
   Gly Gly Val Cys Met His Ile Gly Ser Leu Asp Ser Tyr Thr Cys Asn
   20 25 30
   Cys Val Ile Gly Tyr Ser Gly Asp Arg Cys Gln Thr Arg Asp Leu Arg
   35 40 45
   Trp Trp Glu Leu Arg
   50
<210> 25
   <211> 112
   <212> PRT
   <213> Mus musculus
<400> 25
   Asp Val Leu Met Thr Gln Thr Pro Leu Ser Leu Pro Val Ser Leu Gly
   1 5 10 15
   Asp Gln Ala Ser Ala Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Ile Tyr Tyr Cys Phe Gln Gly
   85 90 95
   Ser His Val Pro Trp Thr Phe Gly Gly Gly Thr Lys Leu Glu Ile Asn
   100 105 110
<210> 26
   <211> 112
   <212> PRT
   <213> Mus musculus
<400> 26
   Asp Val Leu Met Thr Gln Ser Pro Leu Ser Leu Pro Val Thr Pro Gly
   1               5                   10                  15
   Glu Pro Ala Ser Ala Ser Cys Arg Ser Ser Gln Ser Leu Val His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Thr Gly Val Tyr Tyr Cys Phe Gln Gly
                   85                  90                  95
   Ser His Val Pro Trp Thr Phe Gly Gly Gly Thr Lys Val Glu Ile Lys
               100                 105                 110
<210> 27
   <211> 112
   <212> PRT
   <213> Mus musculus
<400> 27
   Asp Val Leu Met Thr Gln Ser Pro Leu Ser Leu Pro Val Thr Pro Gly
   1               5                   10                  15
   Glu Pro Ala Ser Ala Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Thr Gly Ile Tyr Tyr Cys Phe Gln Gly
                   85                  90                  95
   Ser His Val Pro Trp Thr Phe Gly Gly Gly Thr Lys Val Glu Ile Lys
               100                 105                 110
<210> 28
   <211> 112
   <212> PRT
   <213> Mus musculus
<400> 28
   Asp Val Leu Met Thr Gln Ser Pro Leu Ser Leu Pro Val Thr Pro Gly
   1               5                   10                  15
   Glu Pro Ala Ser Ala Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Ile Tyr Tyr Cys Phe Gln Gly
                   85                  90                  95
   Ser His Val Pro Trp Thr Phe Gly Gly Gly Thr Lys Val Glu Ile Lys
               100                 105                 110

## Description

The present invention relates to humanised antibodies and fragments thereof, and in particular, to humanised antibodies specific for the epithelial growth factor receptor (EGFR).

EGFR is a tumour-associated cell surface antigen, and hence a well-known target for antibodies. Durrant *et αl*. (*Prenatal Diagnosis,* 14, 131-140, 1994) describe a mouse monoclonal antibody, known as "340", which binds to EGFR with high specificity. A cell line expressing these antibodies is deposited with the ECACC under accession number 97021428. Monoclonal antibody 340 was raised against the osteosarcoma cell line 791T. Immunoprecipitation studies showed that 340 recognised a membrane glycoprotein of molecular weight 170 kDa from both osteosarcoma tumours and placental tissues. Terminal amino acid sequencing of the purified antigen showed sequence identity to the epidermal growth factor receptor. To confirm that 340 antigen was EGF receptor, radiolabelled EGF and EGF receptor antibodies were shown to bind to the S340 antibody antigen. Furthermore, 340 could compete with EGF binding to its receptor, and EGF could compete with 340 for binding to its antigen. Extensive studies have shown that 340 binds to colorectal, gastric, ovarian, osteosarcoma tumour cell lines. The antibody also recognises foetal trophoblasts and has been used to sort foetal cells from maternal blood.

Several other mouse monoclonal antibodies have been shown to recognise EGF receptor. They fall broadly into two categories: antibodies that bind to the receptor but do not inhibit binding of EGF, and antibodies that bind to the receptor and do inhibit binding of EGF. 340 monoclonal antibodies belong to the latter group.

The use of rodent, especially mouse, monoclonal antibodies for therapeutic and *in vivo* diagnostic applications in man has been found to be limited by immune responses elicited by patients to the rodent antibody. The development of so-called "HAMA" (human anti-mouse antibody) responses in patients has been shown to limit the ability of antibodies to reach their antigenic targets, resulting in reduced effectiveness of the antibodies. In order to reduce the HAMA response, chimaeric antibodies have been developed in which the mouse variable (V) regions are joined to the human constant (C) regions. Such antibodies have proved clinically useful, although the mouse V region component still provides the basis for generating immunogenicity in patients (LoBuglio *et al*., *Proc. Nat. Acad. Sci. USA,* **86,** 4220-4224, 1989). Therefore technology for humanised antibodies has been developed whereby the complementarity determining regions (CDRs) from the rodent antibody are grafted onto human V regions and joined to human C regions, to create antibodies where the only "non-human" components are the CDRs which are adjacent to human framework regions. However, it was soon realised that simple transplantation of the CDRs often resulted in reduced affinity of the humanised antibody and consequently that the introduction of certain non-human amino acids in the human V region framework was required to restore the affinity.

The common aspect of these methods for the production of humanised antibodies is to create antibodies which are essentially non-immunogenic in humans. However, the means by which this is achieved has been by the introduction of as much human sequence as possible into the rodent antibody. It is known that certain short peptide sequences or "epitopes" can be immunogenic in humans. Accordingly, techniques have been developed in which such epitopes are identified by computer analysis and amino acids are replaced to produce non-immunogenic peptides (WO98/52976 and WO00/34317).

The inventors have produced humanised versions of the 340 antibody (known as either as "340Ch" (in the case of the mouse human chimera) or "SC100" (in the case of deimmunised antibodies) with reduced immunogenicity with a view to providing a clinically useful therapeutic tool. Unexpectedly, they have found that certain humanised antibodies showed similar binding to cells expressing EGFR to the original murine antibody but had an increased ability to inhibit the growth of such cells.

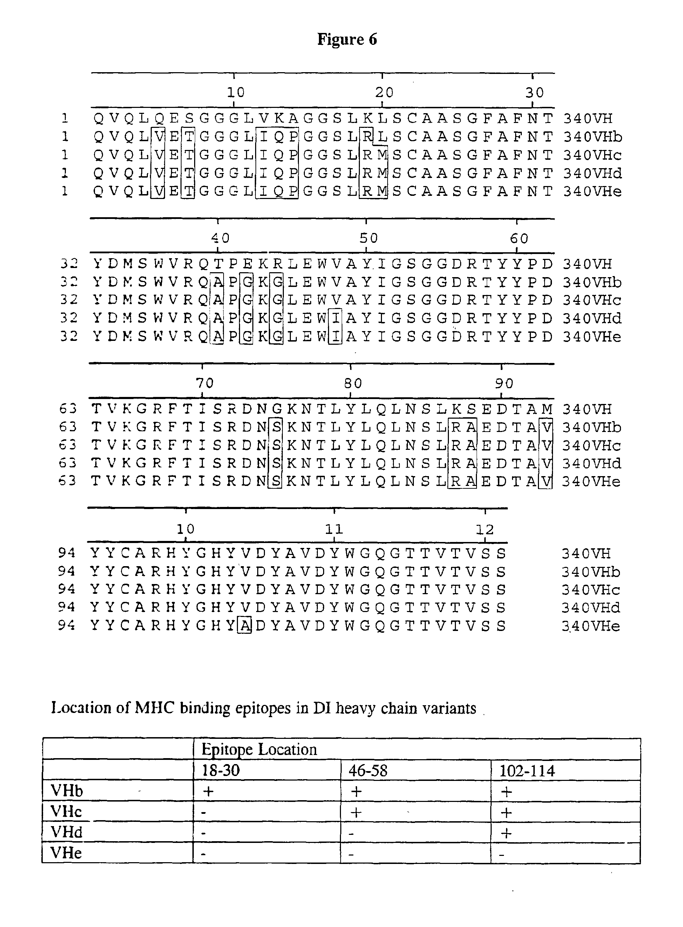

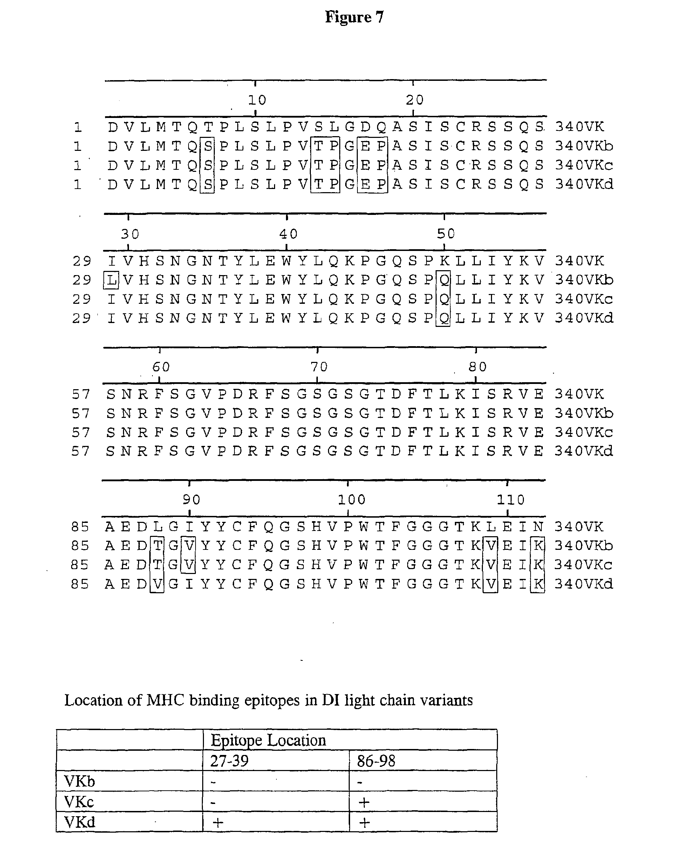

Thus, according to a first aspect of the present invention, there is provided a humanised form of the mouse antibody 340, which mouse antibody is obtainable from the cell line deposited with the ECACC under accession number 97021428, the humanised form comprising the variable region of (i) V_{H}d as shown in Figure 6 and V_{K}d as shown in Figure 7, or (ii) V_{H}d as shown in Figure 6 and V_{K}b as shown in Figure 7.

It is preferred if the antibody of the present invention comprises V_{H}d and V_{K}d or V_{H}d and V_{K}b provided in a human antibody framework. Methods for making such antibodies are known in the art, for example, in Winter, U.S. Patent No. 5,225,539, U.S. Patents Nos. 4,816,397 by Boss (Celltech) and 4,816,567 by Cabilly (Genentech).

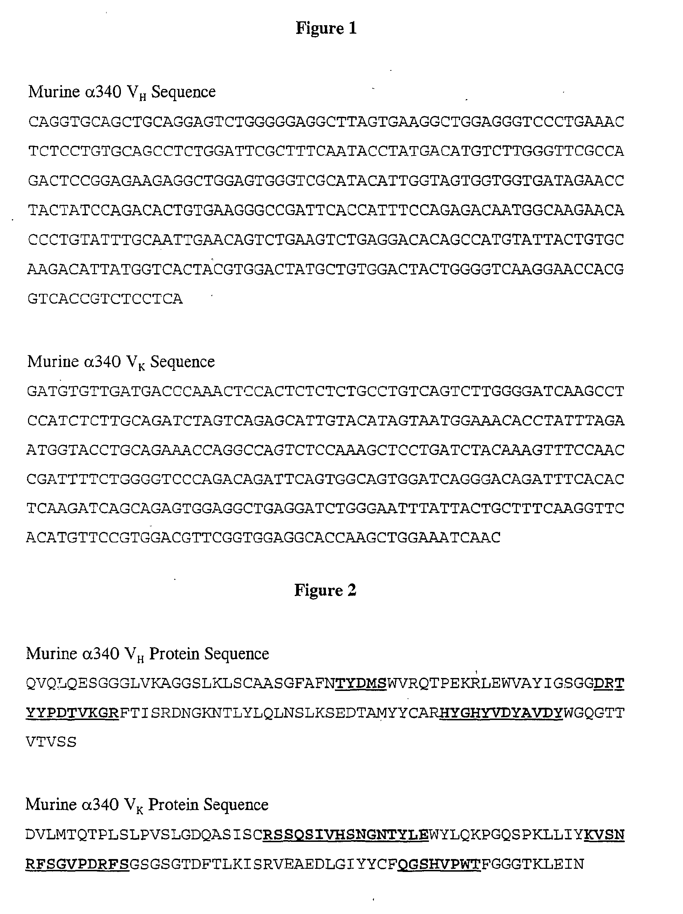

The human antibody framework is preferably the constant region of a human antibody. For example, humanised antibodies based on the V_{K}d or V_{K}b regions shown in Figure 2 may be attached at their C-terminal end to antibody light chain constant domains including human Cκ or Cλ chains. Similarly, antibodies based on the V_{H}d region shown in Figure 2 may be attached at their C-terminal end to all or part of an immunoglobulin heavy chain derived from any antibody isotype, e.g. IgG, IgA, IgE and IgM and any of the isotype sub-classes, particularly IgG1 and IgG4. IgG1 is preferred.

The invention also provides a derivative of an antibody of the first aspect which has 10 or fewer amino acid substitutions and is capable of binding to EGFR and inhibiting the growth of cells at a rate greater than antibody 340

Monoclonal antibodies can block ligands by binding near the ligand binding site of the ligand's receptor and sterically blocking access to the ligand. Alternatively, the antibody may molecularly mimic the ligand and interact at the ligand binding site. The inventors show herein that the 340 antibody and its SC100 derivatives are unique as they not only bind at the ligand binding site but show amino acid homology with two distinct areas of the ligand that are brought together by secondary structure. Furthermore, the importance of the CDRH3 region was confirmed in cell binding studies as, when a single amino acid was changed within this region, binding of SC100 to EGFr was considerably reduced. This implies that an antibody with CDR regions showing homology with peptide ligands can effectively compete with ligand for binding to a receptor. They can therefore block intracellular signalling associated with ligand/receptor interaction. In this example, as EGF is both a growth and a survival factor, blocking its interaction with its receptors results in inhibition of cell growth and apoptosis. As EGF receptors are widely over-expressed in malignancy, blocking EGF receptor by either drug or antibodies inhibits tumour growth. These reagents have been shown to be particularly effective in combination chemotherapy at causing tumour regression in animal models.

As antibodies can be modified in a number of ways, the term "antibody" should be construed as covering any specific binding member or substance having a binding domain with the required specificity. Thus, this term covers antibody fragments, derivatives, functional equivalents and homologues of antibodies, including any polypeptide comprising an immunoglobulin binding domain. The terms "VL", "V_{L}" and "Vₖ" are used interchangeably herein.

It has been shown that fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment (Ward, E.S. *et al*., *Nature* **341:**544-546 (1989)) which consists of a VH domain; (v) isolated CDR regions; (vi) F(ab')2 fragments, a bivalent fragment comprising two linked Fab fragments; (vii) single chain Fv molecules (scFv), wherein a VH domain and a VL domain are linked by a peptide linker which allows the two domains to associate to form an antigen binding site (Bird *et al*., *Science* **242**:423-426 (1988); Huston *et al*., *PNAS USA* **85**:5879-5883 (1988)); (viii) bispecific single chain Fv dimers (PCT/US92/09965) and (ix) "diabodies", multivalent or multispecific fragments constructed by gene fusion (WO94/13804; P. Hollinger *et al*., *Proc. Natl. Acad. Sci. USA* **90**:6444-6448 (1993)).

Diabodies are multimers of polypeptides, each polypeptide comprising a first domain comprising a binding region of an immunoglobulin light chain and a second domain comprising a binding region of an immunoglobulin heavy chain, the two domains being linked (e.g. by a peptide linker) but unable to associated with each other to form an antigen binding site: antigen binding sites are formed by the association of the first domain of one polypeptide within the multimer with the second domain of another polypeptide within the multimer (WO94/13804).

Where bispecific antibodies are to be used, these may be conventional bispecific antibodies, which can be manufactured in a variety of ways (Hollinger & Winter, *Current Opinion Biotechnol.* 1993 **4**:446-449), e.g. prepared chemically or from hybrid hybridomas, or may be any of the bispecific antibody fragments mentioned above. It may be preferable to use scFv dimers or diabodies rather than whole antibodies. Diabodies and scFv can be constructed without an Fc region, using only variable domains, potentially reducing the effects of anti-idiotypic reaction. Other forms of bispecific antibodies include the single chain "Janusins" described in Traunecker *et al*., *EMBO Journal* **10**:3655-3659 (1991).

Bispecific diabodies, as opposed to bispecific whole antibodies, may also be particularly useful because they can be readily constructed and expressed in *E. coli.* Diabodies (and many other polypeptides such as antibody fragments) of appropriate binding specificities can be readily selected using phage display (WO94/13804 from libraries. If one arm of the diabody is to be kept constant, for instance, with a specificity directed against antigen X, then a library can be made where the other arm is varied and an antibody of appropriate specificity selected.

A substantial portion of an immunoglobulin variable domain will comprise at least the three CDR regions, together with their intervening framework regions. Preferably, the portion will also include at least about 50 % of either or both of the first and fourth framework regions, the 50% being the C-terminal 50% of the first framework region and the N-terminal 50% of the fourth framework region. Additional residues at the N-terminal or C-terminal end of the substantial part of the variable domain may be those not normally associated with naturally occurring variable domain regions. For example, construction of antibodies of the present invention made by recombinant DNA techniques may result in the introduction of N- or C-terminal residues encoded by linkers introduced to facilitate cloning or other manipulation steps, including the introduction of linkers to join variable domains of the invention to further protein sequences including immunoglobulin heavy chains, other variable domains (for example in the production of diabodies) or protein labels as discussed in more detail below.

It will be appreciated by those skilled in the art that the sequences of the CDR and variable regions can be modified without losing the ability to bind EGFR. For example, CDR regions of the invention will be either identical or highly homologous to the specified regions of Figures 1 and 2. By "highly homologous" it is contemplated that from 1 to 5, preferably from 1 to 4, such as 1 to 3 or 1 or 2 substitutions may be made in the CDRs. In addition, the variable regions may be modified so that they show substantial homology with the regions specifically disclosed herein. Preferably the degree of homology (between respective CDRs or variable regions and their non-modified counterparts) will be at least 60%, more preferably 70%, further preferably 80%, even more preferably 90% or most preferably 95%. Such modified sequences fall within the scope of the present invention, provided of course that they have the ability to bind EGFR and to inhibit the growth of cells at a greater rate than antibody 340. The term "antibody" is to be construed accordingly.

The percent identity of two amino acid sequences or of two nucleic acid sequences is determined by aligning the sequences for optimal comparison purposes (e.g., gaps can be introduced in the first sequence for best alignment with the sequence) and comparing the amino acid residues or nucleotides at corresponding positions. The "best alignment" is an alignment of two sequences which results in the highest percent identity. The percent identity is determined by the number of identical amino acid residues or nucleotides in the sequences being compared (i.e., % identity = # of identical positions/total # of positions x 100).

The determination of percent identity between two sequences can be accomplished using a mathematical algorithm known to those of skill in the art. An example of a mathematical algorithm for comparing two sequences is the algorithm of Karlin and Altschul (1990) *Proc. Natl*. *Acad. Sci. USA* 87:2264-2268, modified as in Karlin and Altschul (1993) *Proc. Natl*. *Acad. Sci. USA* 90:5873-5877. The NBLAST and XBLAST programs of Altschul, et al. (1990) *J. Mol*. *Biol*. 215:403-410 have incorporated such an algorithm. BLAST nucleotide searches can be performed with the NBLAST program, score = 100, wordlength = 12 to obtain nucleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilised as described in Altschul et al. (1997) *Nucleic Acids Res.* 25:3389-3402. Alternatively, PSI-Blast can be used to perform an iterated search which detects distant relationships between molecules (*Id*.). When utilising BLAST, Gapped BLAST, and PSI-Blast programs, the default parameters of the respective programs (e.g., XBLAST and NBLAST) can be used. *See* http://www.ncbi.nlm.nih.gov.

Another example of a mathematical algorithm utilised for the comparison of sequences is the algorithm of Myers and Miller, CABIOS (1989). The ALIGN program (version 2.0) which is part of the CGC sequence alignment software package has incorporated such an algorithm. Other algorithms for sequence analysis known in the art include ADVANCE and ADAM as described in Torellis and Robotti (1994) *Comput. Appl. Biosci., 10* :3-5; and FASTA described in Pearson and Lipman (1988) *Proc. Natl. Acad. Sci.* 85:2444-8. Within FASTA, ktup is a control option that sets the sensitivity and speed of the search.

As shown herein, the CDRH3 of SC100 shows amino acid homology with the growth factor EGF. These results described below suggest that SC100 and fragments and derivatives thereof can be used as cancer therapeutics to inhibit the growth or induce apoptosis of tumour cells as exemplified by inhibition of growth of tumour cell lines, apoptosis of tumour cell lines, *in vivo* inhibition of tumour xenografts in nude mice. Accordingly, the invention further includes the use of "fragments" or "derivatives" of either SC 100 or other polypeptides of the "SC100" family which are capable of inhibition of binding of EGF. A preferred group of fragments are those which include all or part of the CDR regions of monoclonal antibodies SC100. A fragment of SC100 or of a polypeptide of the SC100 families means a stretch of amino acid residues of at least 5 to 7 contiguous amino acids. Often at least about 7 to 9 contiguous amino acids, typically at least about 9 to 13 contiguous amino acids, more preferably at least about 20 to 30 or more contiguous amino acids and most preferably at least about 30 to 40 or more consecutive amino acids. A "derivative" of SC100 or of a polypeptide of the SC100 family, or of a fragment of SC100 family polypeptide, means a polypeptide modified by varying the amino acid sequence of the protein, e.g. by manipulation of the nucleic acid encoding the protein or by altering the protein itself. Such derivatives of the natural amino acid sequence may involve insertion, addition, deletion and/or substitution of one or more amino acids, while providing a peptide capable of inducing an antitumour T-cell response. Preferably such derivatives involve the insertion, addition, deletion and/or substitution of 25 or fewer amino acids, more preferably of 15 or fewer, even more preferably of 10 or fewer, more preferably still of 4 or fewer and most preferably of 1 or 2 amino acids only.

The invention also provides the antibodies mentioned above linked to a coupling partner, e.g. an effector molecule, a label, a drug, a toxin and/or a carrier or transport molecule. Techniques for coupling the antibodies of the invention to both peptidyl and non-peptidyl coupling partners are well known in the art. In one embodiment, the carrier molecule is a 16 amino acid peptide derived from the homeodomain of *Antennapedia* (e.g. as sold under the name "Penetratin"), which can be coupled to a peptide via terminal Cys residue. The "Penetratin" molecule and its properties are described in WO 91/18981.

Thus, antibodies of the invention may be labelled with a detectable label, for example a radiolabel such as ¹³¹I or ⁹⁹Tc, which may be attached to using conventional chemistry known in the art of antibody imaging. Labels also include enzyme labels such as horseradish peroxidase. Labels further include chemical moieties such as biotin which may be detected via binding to a specific cognate detectable moicty, e.g. labelled avidin. Therefore, antibodies of the present invention may be labelled with a functional label. Such functional labels include toxins such as ricin and enzymes such as bacterial carboxypeptidase or nitroreductase, which are capable of converting prodrugs into active drugs at the site of cancer.

The antibodies of the present invention may be generated wholly or partly by chemical synthesis. The antibodies of the present invention can be readily prepared according to well-established, standard liquid or, preferably, solid-phase peptide synthesis methods, general descriptions of which are broadly available (see, for example, in J.M. Stewart and J.D. Young, Solid Phase Peptide Synthesis, 2^{nd} edition, Pierce Chemical Company, Rockford, Illinois (1984), in M. Bodanzsky and A. Bodanzsky, The Practice of Peptide Synthesis, Springer Verlag, New York (1984); and Applied Biosystems 430A Users Manual, ABI Inc., Foster City, California), or they may be prepared in solution, by the liquid phase method or by any combination of solid-phase, liquid phase and solution chemistry, e.g. by first completing the respective peptide portion and then, if desired and appropriate, after removal of any protecting groups being present, by introduction of the residue X by reaction of the respective carbonic or sulphonic acid or a reactive derivative thereof.

Another convenient way of producing an antibody according to the present invention (peptide or polypeptide) is to express nucleic acid encoding it, by use of nucleic acid in an expression system.

Accordingly, the present invention also provides nucleic acid encoding the antibodies of the invention.

Generally, nucleic acid according to the present invention is provided as an isolate, in isolated and/or purified form, or free or substantially free of material with which it is naturally associated, such as free or substantially free of nucleic acid flanking the gene in the human genome, except possibly one or more regulatory sequence(s) for expression. Nucleic acid may be wholly or partially synthetic and may include genomic DNA, cDNA or RNA. Where nucleic acid according to the invention includes RNA, reference to the sequence shown should be construed as reference to the RNA equivalent, with U substituted for T.

Nucleic acid sequences encoding a polypeptide or peptide in accordance with the present invention can be readily prepared by the skilled person using the information and references contained herein and techniques known in the art (for example, see Sambrook, Fritsch and Maniatis, "Molecular Cloning" , A Laboratory Manual, Cold Spring Harbor Laboratory Press, 1989, and Ausubel et al, Short Protocols in Molecular Biology, John Wiley and Sons, 1992), given the nucleic acid sequences and clones available. These techniques include (i) the use of the polymerase chain reaction (PCR) to amplify samples of such nucleic acid, e.g. from genomic sources, (ii) chemical synthesis, or (iii) preparing cDNA sequences. DNA encoding SC100 fragments may be generated and used in any suitable way known to those of skill in the art, including by taking encoding DNA, identifying suitable restriction enzyme recognition sites either side of the portion to be expressed, and cutting out said portion from the DNA. The portion may then be operably linked to a suitable promoter in a standard commercially available expression system. Another recombinant approach is to amplify the relevant portion of the DNA with suitable PCR primers. Modifications to the sequences can be made, e.g. using site directed mutagenesis, to lead to the expression of modified peptide or to take account of codon preferences in the host cells used to express the nucleic acid.

In order to obtain expression of the nucleic acid sequences, the sequences can be incorporated into a vector having one or more control sequences operably linked to the nucleic acid to control its expression. The vectors may include other sequences such as promoters or enhancers to drive the expression of the inserted nucleic acid, nucleic acid sequences so that the polypeptide or peptide is produced as a fusion and/or nucleic acid encoding secretion signals so that the polypeptide produced in the host cell is secreted from the cell. Polypeptide can then be obtained by transforming the vectors into host cells in which the vector is functional, culturing the host cells so that the polypeptide is produced and recovering the polypeptide from the host cells or the surrounding medium. Prokaryotic and eukaryotic cells are used for this purpose in the art, including strains of *E. coli,* yeast, and eukaryotic cells such as COS or CHO cells.

Thus, the present invention also encompasses a method of making an antibody of the present invention, the method including expression from nucleic acid encoding the antibody (generally nucleic acid according to the invention). This may conveniently be achieved by growing a host cell in culture under appropriate conditions which cause or allow expression of the antibody.

Polypeptides and peptides may also be expressed in *in vitro* systems, such as reticulocyte lysate.

Systems for cloning and expression of a polypeptide in a variety of different host cells are well known. Suitable host cells include bacteria, eukaryotic cells such as mammalian and yeast, and baculovirus systems. Mammalian cell lines available in the art for expression of a heterologous polypeptide include Chinese hamster ovary cells, HeLa cells, baby hamster kidney cells, COS cells and many others. A common, preferred bacterial host is *E*. *coli.*

Suitable vectors can be chosen or constructed, containing appropriate regulatory sequences, including promoter sequences, terminator fragments, polyadenylation sequences, enhancer sequences, marker genes and other sequences as appropriate. Vectors may be plasmids, viral e.g. 'phage, or phagemid, as appropriate. For further details see, for example, Molecular Cloning: a Laboratory Manuel: 2^{nd} edition, Sambrook et al., 1989, Cold Spring Harbor Laboratory Press. Many techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Ausubel et al. eds., John Wiley and Sons, 1992.

Thus, a further aspect of the present invention provides a host cell containing heterologous nucleic acid as disclosed herein.

The nucleic acid of the invention may be integrated into the genome (e.g. chromosome) of the host cell. Integration may be promoted by inclusion of sequences which promote recombination with the genome in accordance with standard techniques. The nucleic acid may be on an extra-chromosomal vector within the cell, or otherwise identifiably heterologous or foreign to the cell.

A still further aspect of the invention provides a method which includes introducing the nucleic acid into a host cell. The introduction, which may (particularly for *in vitro* introduction) be generally referred to without limitation as "transformation", may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. For bacterial cells, suitable techniques may include calcium chloride transformation, electroporation and transfection using bacteriophage. As an alternative, direct injection of the nucleic acid could be employed.

Marker genes such as antibiotic resistance or sensitivity genes may be used in identifying clones containing nucleic acid of interest, as is well known in the art.

The introduction may be followed by causing or allowing expression from the nucleic acid, e.g. by culturing host cells (which may include cells actually transformed although more likely the cells will be descendants of the transformed cells) under conditions for expression of the gene, so that the encoded polypeptide (or peptide) is produced. If the polypeptide is expressed coupled to an appropriate signal leader peptide it may be secreted from the cell into the culture medium. Following production by expression, a polypeptide or peptide may be isolated and/or purified from the host cell and/or culture medium, as the case may be, and subsequently used as desired, e.g. in the formulation of a composition which may include one or more additional components, such as a pharmaceutical composition which includes one or more pharmaceutically acceptable excipients, vehicles or carriers (e.g. see below).

As mentioned previously, the polypeptide may also be conjugated to an effector molecule. The effector molecule performs various useful,functions such as, for example, inhibiting tumour growth, permitting the polypeptide to enter a cell such as a tumour cell, and directing the polypeptide to the appropriate location within a cell.

The effector molecule, for example, may be a cytotoxic molecule. The cytotoxic molecule may be a protein, or a non-protein organic chemotherapeutic agent. Some examples of suitable chemotherapeutic agents include, for example, doxorubicin, taxol and cisplatin.

Some additional examples of effector molecules suitable for conjugation to the polypeptides of the invention include signal transduction inhibitors, ras inhibitors, and cell cycle inhibitors. Some examples of signal transduction inhibitors include protein tyrosine kinase inhibitors, such as quercetin (Grazieri *et al*., *Biochim. Biophs. Acta* **714**, 415 (1981)); lavendustin A *(Onoda et al*., *J. Nat. Produc.* **52**, 1252 (1989)); and herbimycin A *(Ushara et al*., *Biochem. Int.,* **41**: 831 (1988)).

Proteins and non-protein chemotherapeutic agents may be conjugated to the antibodies of the invention by methods that are known in the art. Such methods include, for example, that described by Greenfield *et al*., *Cancer Research 50,* 6600-6607 (1990) for the conjugation of doxorubicin and those described by *Arnon et al*., Adv. Exp. *Med. Biol.* **303**, 79-90 (1991) and by Kiseleva *et al*., *Mol. Biol.* (USSR) 25, 508-514 (1991) for the conjugation of platinum compounds. Doxorubicin, taxol and cisplatin are preferred.

The antibodies of the invention can be formulated in pharmaceutical compositions with a pharmaceutically acceptable carrier. These compositions may comprise, in addition to one of the above substances, a pharmaceutically acceptable excipient, buffer, stabiliser or other materials well known to those skilled in the art. Such materials should be non-toxic and should not interfere with the efficacy of the active ingredient. The precise nature of the carrier or other material may depend on the route of administration, e.g. oral, intravenous, cutaneous or subcutaneous, nasal, intramuscular, intraperitoneal routes. The formulation is preferably liquid, and is ordinarily a physiologic salt solution containing non-phosphate buffer at pH 6.8-7.6, or may be lyophilised powder.

The compositions comprising or for the delivery of the antibodies of the present invention are preferably administered to an individual in a "therapeutically effective amount", this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners.

The antibodies of the invention are particularly relevant to the treatment of existing cancer and in the prevention of the recurrence of cancer after initial treatment or surgery. Examples of the techniques and protocols mentioned above can be found in Remington's Pharmaceutical Sciences, 16^{th} edition, Oslo, A. (ed), 1980. Thus, the invention also provides (a) an antibody or nucleic acid of the invention for use in medicine, and the use of an antibody or nucleic acid of the invention in the manufacture of a medicament for the treatment or prophylaxis of cancer.

The antibodies of the invention can significantly inhibit the growth of tumour cells when administered to a human in an effective amount. The optimal dose can be determined by physicians based on a number of parameters including, for example, age, sex, weight, severity of the condition being treated, the active ingredient being administered and the route of administration. In general, a serum concentration of polypeptides and antibodies that permits saturation of EGF receptors is desirable. A concentration in excess of approximately 0.1 nM is normally sufficient. For example, a dose of 100mg/m² of antibody provides a serum concentration of approximately 20nM for approximately eight days.

As a rough guideline, doses of antibodies may be given weekly in amounts of 10-300mg/m². Equivalent doses of antibody fragments should be used at more frequent intervals in order to maintain a serum level in excess of the concentration that permits saturation of EGF receptors.

Some suitable routes of administration include intravenous, subcutaneous and intramuscle administration. Intravenous administration is preferred.

The antibodies of the invention may be administered along with additional pharmaceutically acceptable ingredients. Such ingredients include, for example, immune system stimulators and chemotherapeutic agents, such as those mentioned above.

A composition may be administered alone or in combination with other treatments, either separately, simultaneously or sequentially, dependent upon the condition to be treated. Other cancer treatments include other monoclonal antibodies, other chemotherapeutic agents, other radiotherapy techniques or other immuno therapy known in the art. One particular application of the compositions of the invention are as an adjunct to surgery, i.e. to help to reduce the risk of cancer reoccurring after a tumour is removed.

It is envisaged that injections (iv) will be the primary route for therapeutic administration of the antibodies of this invention, intravenous delivery, or delivery through a catheter or other surgical tubing is also used. Liquid formulations may be utilised after reconstitution from powder formulations.

The antibody may also be administered via microspheres, liposomes, other microparticulate delivery systems or sustained release formulations placed in certain tissues including blood. Suitable examples of sustained release carriers include semipermeable polymer matrices in the form of shared articles, e.g. suppositories or microcapsules. Implantable or microcapsular sustained release matrices include polylactides (US Patent No. 3, 773, 919, EP-A-0058481) copolymers of L-glutamic acid and gamma ethyl-L-glutamate (Sidman *et al*, *Biopolymers* **22**(1): 547-556, 1985), poly (2-hydroxyethyl-methacrylate) or ethylene vinyl acetate (Langer *et al*, *J. Biomed. Mater. Res.* **15:** 167-277, 1981, and Langer, *Chem*. *Tech.* **12**:98-105, 1982). Liposomes containing the polypeptides are prepared by well-known methods: DE 3,218, 121A; Epstein et al, PNAS USA, 82: 3688-3692, 1985; Hwang et al, PNAS USA, 77: 4030-4034, 1980; EP-A-0052522; E-A-0036676; EP-A-0088046; EP-A-0143949; EP-A-0142541; JP-A-83-11808; US Patent Nos 4,485,045 and 4,544,545. Ordinarily, the liposomes are of the small (about 200-800 Angstroms) unilamellar type in which the lipid content is greater than about 30 mol. % cholesterol, the selected proportion being adjusted for the optimal rate of the polypeptide leakage.

The antibodies of the present invention may be administered in a localised manner to a tumour site or other desired site or may be delivered in a manner in which it targets tumour or other cells.

The dose of antibodies will be dependent upon the properties of the agent employed, e.g. its binding activity and *in vivo* plasma half-life, the concentration of the polypeptide in the formulation, the administration route, the site and rate of dosage, the clinical tolerance of the patient involved, the pathological condition afflicting the patient and the like, as is well within the skill of the physician. For example, doses of 300µg of polypeptide per patient per administration are preferred, although dosages may range from about 10µg to 1 mg per dose. Different dosages are utilised during a series of sequential inoculations; the practitioner may administer an initial inoculation and then boost with relatively smaller doses of antibody.

The antibodies of the invention can be administered in a variety of ways and to different classes of recipients. Examples of types of cancer that can be treated with the antibody include colorectal cancer, lung , breast, gastric and ovarian cancers.

This invention is also directed to optimise immunisation schedules for enhancing a protective immune response against cancer.

Preferred features of each aspect of the present invention are as for each other aspect *mutatis mutandis.*

The invention will be described further with reference to the following non-limiting examples. Reference is made to the accompanying drawings in which:
Figure 1 shows the DNA sequences of the mouse monoclonal antibody 340 Vₕ and
Hₖ sequences;
Figure 2 shows the translated protein sequence of the mouse monoclonal antibody 340 V_{H} and Vₖ sequences. The emboldened and underlined sequences represent the CDRs which are sequential, i.e. CDR1 is the first sequence shown and CDR3 is the last sequence shown for each chain;
Figure3 shows the sequences of primers used to amplify murine Vₕ and Vₖ sequences;
Figure 4 is a diagrammatic representation of an expression vector used for expression of a heavy chain;
Figure 5 is a diagrammatic representation of an expression vector used for expression of a light chain;
Figure 6 shows the alignment of deimmunised heavy chain sequences, including the location of the MHC binding epitopes in the deimmunised heavy chain variants;
Figure 7 shows the alignment of deimmunised light chain sequences including the location of the MHC binding epitopes in the deimmunised light chain;
Figure 8 shows the results of an A431 cell binding assay with a340 chimerised and a340 mouse monoclonal antibodies;
Figure 9 shows the results of an A431 cell binding assay of Vhd deimmunised variants;
Figure 10 shows the results of an A431 cell binding assay of Vhe deimmunised variants;
Figure 11 shows the results of an A431 cell binding assay of Vhb deimmunised variants;

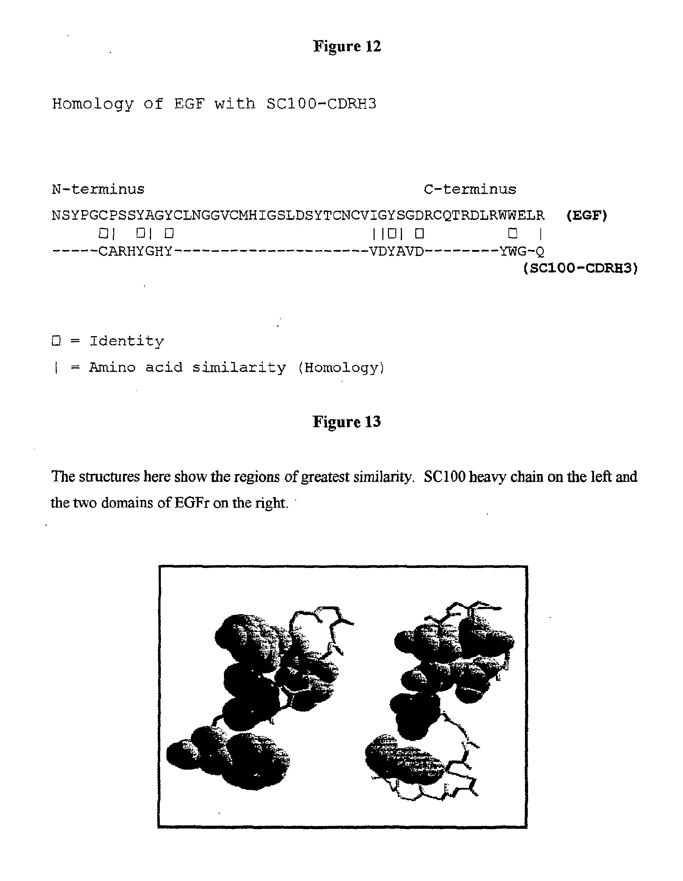

Figure12 illustrates the amino acid homology between the SC100 Monoclonal Antibody and EGF. The amino acid sequence deduced for the complementarily determining region 3 of the immunoglobulin heavy chain of SC100 antibody shows distinct homology with specific areas of the published sequence for Epidermal Growth Factor;
Figure 13 is a molecular model comparing EGF and SC100. These illustrations represent SC100 Heavy chain and EGF respectively and illustrate the structural similarity between the two regions of amino acid homology represented in Figure 6. In particular, these diagrams show how the two areas of similar sequence are brought into close proximity in the EGF structure and how they then are mimicked by the homologous sequence in the CDRH3 of SC100 antibody; and

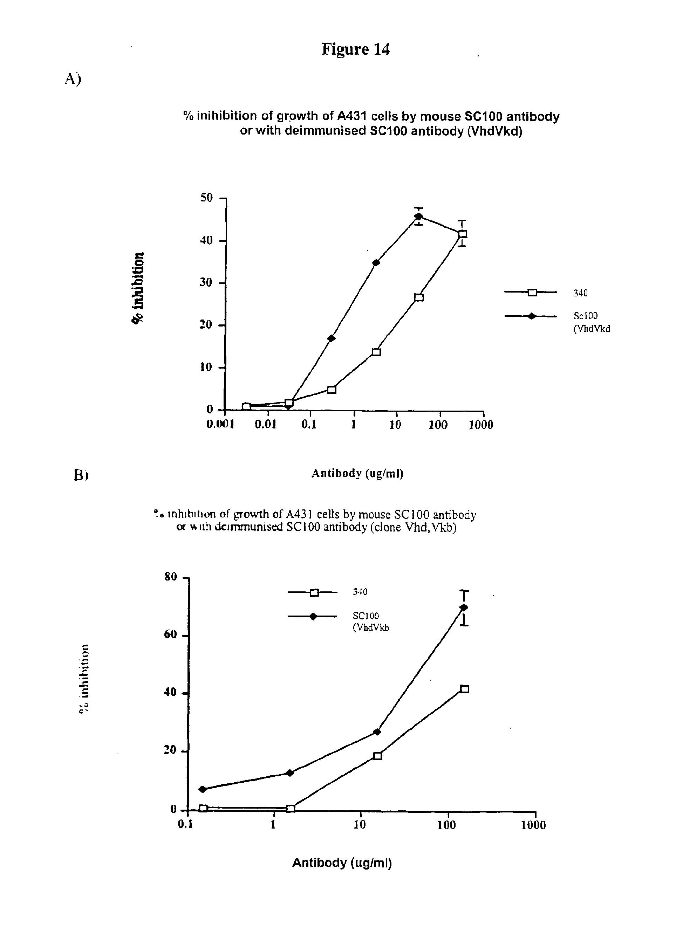

Figure 14a shows the % inhibition of growth of A431 cells for deimmunised SC100 antibody (clone VhdVkd) (a) and for deimmunised SC100 (clone VhdVkb) (b) compared with mouse 340 monoclonal antibody.

### EXAMPLES

### Example 1- Construction of Chimaeric Antibody derived from α340

Total RNA was isolated from 5 x 10⁶ hybridoma α340 cells (Durrant *et al*., *Prenatal Diagnostics,* **14**, 131, 1994) using Qiagen RNeasy kit following manufacturers instructions. The RNA was converted into cDNA using Promega (Southampton, UK) reverse transcriptase, buffer and dNTPs. Variable region heavy (V_{H}) and light (V_{L}) chain cDNAs were amplified using primer sets of the method of Jones and Bendig *(Bio*/*Technology,* **9,** 188, 1991). The amplified DNAs were gel-purified and cloned into the vector pGem® T Easy (Promega). These PCR products were sequenced in both directions using the Applied Biosystems automated sequencer model 373A (Applied Biosystems, Warrington, UK). Resultant V_{H} and V_{L} DNA sequences are shown in Figure 1 and the protein sequences in Figure 2 (as used herein V_{L} is the same as V_{K}).

The location of the complementarity determining regions (CDRs) was determined with reference to other antibody sequences (Kabat EA *et al*., US Department of Health and Human Services, 1991). The α340 V_{H} can be assigned to Mouse Heavy Chains Subgroup III(B) (Kabat *et al*., 1991) and α340 V_{K} can be assigned to Mouse Kappa Chains Subgroup II (Kabat *et al*, 1991).

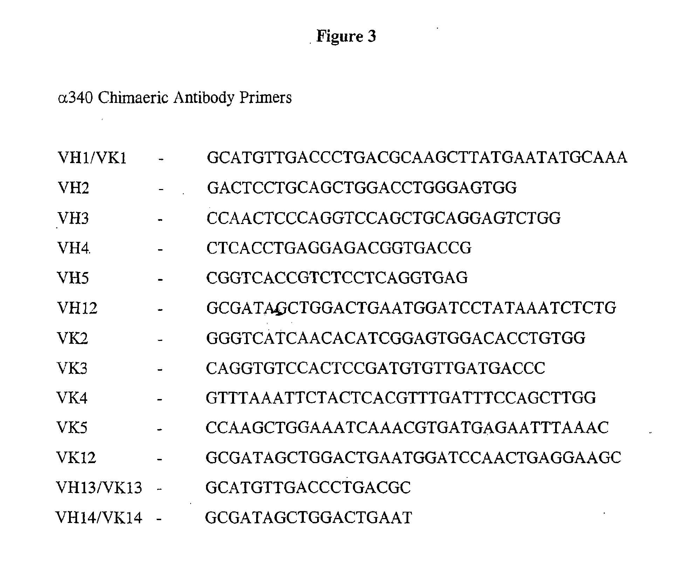

The chimaeric antibody consists of murine variable regions linked to human constant regions. The chimaeric antibody also provides a useful control with the same human constant regions when testing the DeImmunised antibodies (see below). The vectors V_{H}-PCR1 and V_{K}-PCR1 (Riechmann *et al*., Nature, **332,** 323, 1988) were used as templates to introduce 5' flanking sequence including the leader signal peptide, leader intron and the murine immunoglobulin promoter, and 3' flanking sequence including the splice site and intron sequences, around the murine V_{H} and V_{K} genes. The murine V_{H} and V_{K} sequences were amplified by primers overlapping with the V_{H}/V_{K}-PCR1 vector sequences (see Figure 3) using *pfu* proof-reading polymerase (*pfu* turbo, Stratagene, La Jolla, California). This enabled construction of the full-length chimaeric heavy and light chains. These PCR primers were used to amplify the V_{H}/V_{K} regions and the 5' and 3' human regions from the V_{H}/V_{K}PCR-1 vectors.

The 5' Hu_{H/K}, V_{H/K} and 3' Hu_{H/K} regions were connected and amplified using flanking primers (VH/K13, VH/K14) recognising the ends of VH/K1 and VH/K12 PCR primers giving a complete chimaeric antibody expression cassette. This product was cleaved with BamHI and HindIII restriction enzymes and ligated into BamHI/HindIII cut pUC19 (Enzymes by Promega, pUC19 by Amersham Pharmacia). The sequence of the expression cassette was then confirmed by sequencing as described previously.

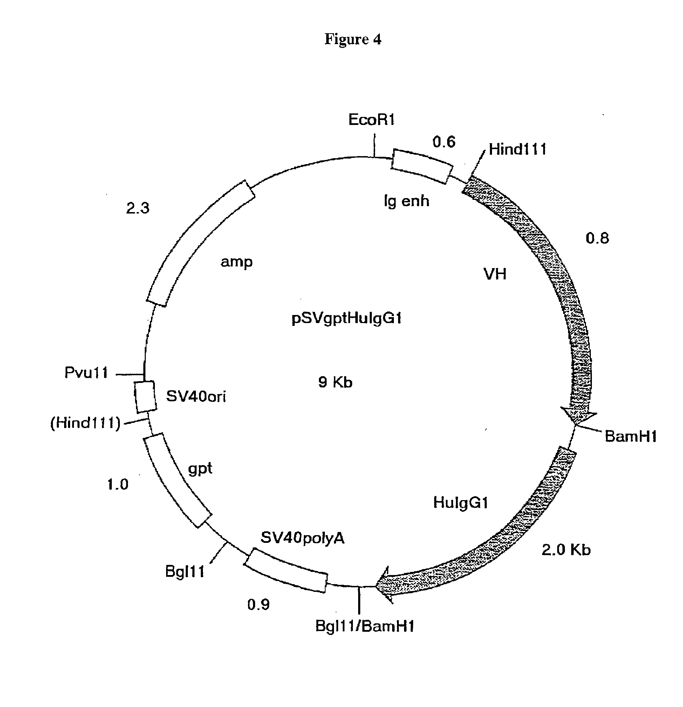

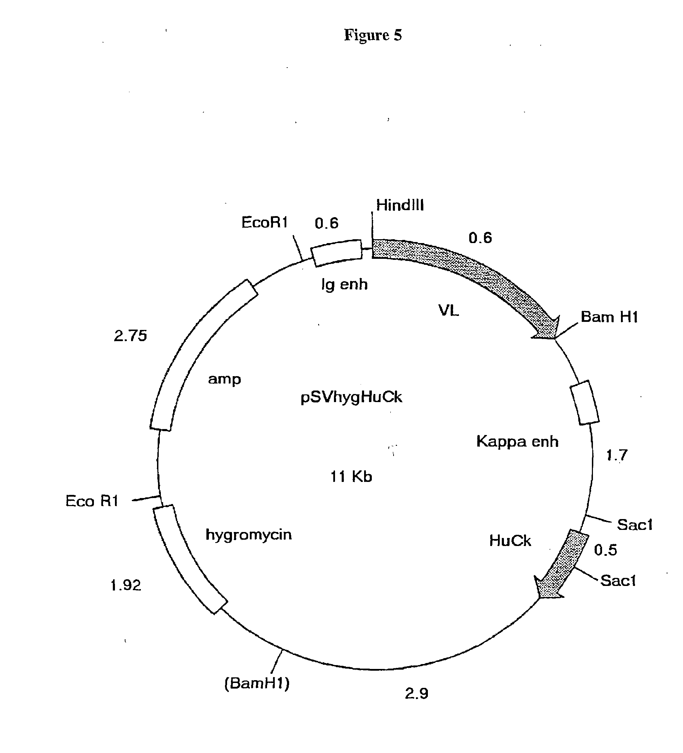

The murine V_{H} and V_{L} expression cassettes were excised from pUC19 as *Hin*dIII to *Bam*HI fragments, which include the murine heavy chain immunoglobulin promoter, the leader signal peptide, leader intron, the V_{H} or V_{L} sequence and the splice site. These were transferred to the expression vectors pSV*gpt* and pSV*hyg* (Figures 4 and 5), which include human IgG1 or κ constant regions respectively and markers for selection in mammalian cells. The DNA sequence was confirmed to be correct for the V_{H} and V_{L} in the expression vectors.

### Example 2 - Design of α340 DeImmunised Sequences

The following example describes the method by which the human immune response elicited by a pre-existing therapeutic antibody is reduced. There are two steps by which this was achieved, initially the murine heavy and light chain sequences were compared to a database of human germ-line sequences. The most similar germ-line sequences were chosen as the human template for the deimmunised sequence and changes necessary to convert the murine to the human germ-line sequence were introduced. Residues which were considered to be critical for antibody structure and binding were excluded from this process and not altered. The murine residues that were retained at this stage were largely non-surface, buried residues, apart from residues at the N-terminus for instance, which are close to the CDRs in the final antibody. This process produces a sequence that is broadly similar to a "veneered" antibody as the surface residues are mainly human and the buried residues are as in the original murine sequence.

In the current example, the variable region protein sequences for the α340 antibody have been individually compared to the human germ-line V_{H/K} sequences. In the case of α340, the α340V_{H} chain was seen to be most similar to germ-line sequences V_{H}DP42 and J_{H}6. The α340V_{K} chain showed most similarity with V_{K}DP15 and J_{K}2.

The second step involves the identification of antibody V_{H/K} epitopes responsible for the immune response, and modification of the antibody sequence to remove such sequences. α340 was analysed by a novel process of peptide threading. Analysis was conducted by computer using MPT ver1.0 software (Biovation, Aberdeen, UK). This software package conducts peptide threading according to the methods disclosed in WO98/52976. The software is able to provide an index of potential peptide binding to 18 different MHC class II DR alleles covering greater than 96% of the HLA DR allotypes extant in the human population.

The α340 antibody chains, designed to mimic the human germ-line sequences described previously, were threaded by this method and potential MHC class II epitopes identified. Such epitopes were mutated by substitutions of amino acids responsible for MHC class II binding, by similar residues. These substitutions are designed to maintain general antibody structure and antigen binding capacity but remove the MHC epitope.

Several variants of each chain were designed to give a range of antibodies with differing levels of MHC binding chains and mutations from the original murine α340. Three DIV_{*K*} and four DIV_{*H*} variants were designed (V_{*K*}b, V_{*K*}c, V_{*K*}d, V_{*H*}b, V_{H}c, V_{H}d and V_{H}e) and can be seen in the respective alignments to show the MHC epitopes and mutations introduced to remove them (see Figures 6 and 7).

The mutations introduced include two in the CDRs of the α340 antibody. The I-L mutation of V_{K}b is contained in CDR1 of the V_{K} region. The V-A mutation of V_{H}e is similarly contained in the CDR3 of the V_{H} region. These substitutions could have a considerable effect on the antigen binding capacity of the α340 antibody and illustrate the importance of producing other variants with differing mutations.

### Example 3 - Construction of DeInimunised Antibody Sequences

The DeImmunised variable regions were constructed by the method of overlapping PCR recombination. The cloned murine V_{H} and V_{K} genes were used as templates for mutagenesis of the framework regions to the required DeImmunised sequences. Sets of mutagenic primer pairs were synthesised encompassing the regions to be altered.

The use of mutagenic primer pairs dictated the use of annealing temperatures of 48°C-50°C. *pfu* turbo proof-reading polymerase (Stratagene, La Jolla, California) was used for all amplifications. The α340 chimaeric V_{H} and V_{K} constructs were used as templates to introduce 5' flanking sequence including the leader signal peptide, leader intron and the murine immunoglobulin promoter, and 3' flanking sequence including the splice site and intron sequences as well as the variable regions to be modified. Overlapping PCRs were performed using the same flanking primers (VH/K13, VH/K14) as to create the chimaeric expression cassette. The DeImmunised V regions produced were cloned into pUC19 and the entire DNA sequence was confirmed to be correct for each DeImmunised V_{H} and V_{L}.

The DeImmunised heavy and light chain V-region genes were excised from pUC19 as *Hin*dIII to *Bam*HI fragments, which include the murine heavy chain immunoglobulin promoter, the leader signal peptide, leader intron, the V_{H} or V_{L} sequence and the splice site. These were transferred to the expression vectors pSV*gpt* and pSV*hyg*, which include human IgG1 or κ constant regions respectively and markers for selection in mammalian cells. The DNA sequence was confirmed to be correct for the Delmmunised V_{H} and V_{L} in the expression vectors. To prepare the constructs for transformation, approximately 50µg of V_{K} and 25µg of V_{H} plasmid (per transformation) was digested with pvuI (Promega, Southampton, UK) to completion. This DNA was then ethanol precipitated and the DNA pellet dried. Prior to transformation the pellet was resuspended in 10µl of molecular biology grade water (per transformation).

### Example 4 - Expression of α340 DeImmunised Antibodies

The host cell line for antibody expression was NSO, a non-immunoglobulin producing mouse myeloma, obtained from the European Collection of Animal Cell Cultures, Porton, UK (ECACC No 85110505). The heavy and light chain expression vectors were co-transfected in a variety of combinations into NS0 cells by electroporation. Each DIV_{H} chain was transfected with each DIV_{K} chain to give 12 deinununised variant antibodies. In addition, the chimaeric V_{H} and V_{K} regions were transfected to produce a cell line expressing the chimaeric α340 antibody (α340Ch). The α340Ch antibody was used as a control to show the binding of the antibody before deimmunisation.

NS0 cells were grown in a 75cm³ flask (NalgeNunc Int., Rochester, NY, USA) in 20mls of Dulbecco's Modified Eagle's Medium (DMEM) supplemented with 10% foetal bovine serum (FBS), 5ml antibiotics/antimycotics solution (Gibco BRL, Paisley, UK. Cat no. 15240-062), 2.5mls gentamicin (Gibco BRL, Paisley, UK. Cat no. 15710-049) and 5mls sodium pyruvate (Gibco BRL, Paisley, UK. Cat no. 11360-039) per 500mls of media. Once confluent, these cells were centrifuged into a pellet and resuspended in 0.5mls (per transformation) of identical media. This 0.5ml of cells was then added to the DNA previously digested, precipitated and resuspended and incubated on ice for 5 minutes. The cells were then aliquoted into a 2mm cuvette (Biorad, Hercules, CA, USA) and pulsed at 170 volts and 975µF in a Biorad Genepulser II, and then placed on ice for 20 minutes to recover. The cells were then aliquoted into 20mls of DMEM/10%FBS as described above and grown overnight at 37°C, 5% CO₂.

Twenty-four hours later, the cells were centrifuged and resuspended in 85mls of selective DMEM/10%FBS (as described plus 5mls of 25mg/ml xanthine and 160µl of 2.5mg/ml mycophenolic acid per 500mls of media. These are selective agents for the *gpt* gene of the psv heavy chain vector). This was then aliquoted into 4 x 96 well plates in 200µl aliquots per well. These plates were grown for 10 days, until resistant colonies developed.

Production of human antibody by transfected cell clones was measured by ELISA for human IgG. Cell lines secreting antibody were selected and expanded, initially into 24 well plates. These were then expanded up into 25cm³ flasks and 75cm³ flasks. Antibody production was assayed by ELISA by comparison with known concentrations of human control antibody. The best antibody producing clones from each transfection were then expanded into 175cm³ flasks and the other clones frozen down in liquid nitrogen.

No antibody was produced from cells transfected with V_{H} version C. It is unclear as to why but considerable numbers of transfectant colonies were produced on three separate occasions and no antibody producing colonies found. The production of variants carrying V_{H}b, V_{H}d and V_{H}e was continued as described, but V_{H}c was not continued.

### Example 5 - Production and testing of DeImmunised α340 antibodies

Antibody was purified from 500 ml to 1 litre static cultures by stirring with 0.5ml of ProSepA (Bioprocessing Ltd) overnight. The Prosep A was then isolated by affinity chromatography. Antibody was eluted with 0.1M glycine pH3.0, neutralised and dialysed against PBS overnight. Purified antibody preparations were filter sterilised by filtration and stored at 4°C. The concentration of purified antibody was determined by ELISA for Human IgG.

The α340 DI antibody variants were tested in an ELISA for binding to A431 cells (ECCAC No. 85090402). These epithelial monolayer cells over express the epithelial growth factor receptor (EGFR) and are therefore suitable for assaying binding of α340 to the EGFR antigen. A431 cells were grown to confluence in 96 well plates in DMEM/10% FBS media and 1% non-essential amino acids (NEAA, Gibco BRL, Cat no. 11140-035). The media was then removed and the cells washed 3 times in PBS. They were then incubated in Immunoassay Stabiliser (Quadratech) for 1 hour at room temperature. The solution was then discarded and the plates left to dry for at room temperature. Plates were then stored at -4°C.

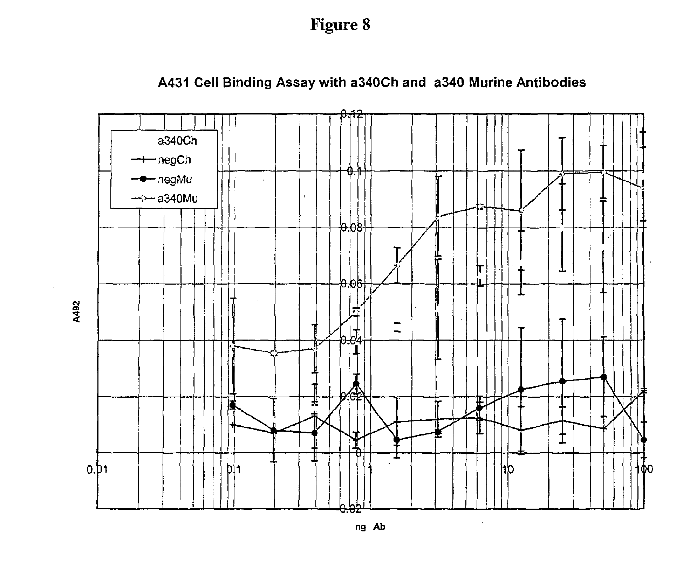

Assays comparing the α340Ch antibody with the original murine α340 showed comparable binding of the chimaeric form (Figure 8). Washes were performed with PBS with 0.05% Tween (Sigma). Detection was with horseradish peroxidase conjugated goat anti-human IgG (The Binding Site, Cambridge, UK) and sheep anti-mouse (The Binding Site, Cambridge, UK) for chimaeric and mouse antibodies respectively. Colour was developed with o-phenylene diamine substrate (Sigma, Poole, Dorset, UK).

The results are not directly comparable due to the use of different secondary antibodies with potentially differing binding capacities. However, this clearly showed that α340Ch bound the EGFR of A431 cells and could be used as a positive control to compare the binding affinities of the DI α340 variants.

To assay the DI α340 variants, doubling dilutions (from 100ng per well) of the DI variants and α340 chimaeric antibody (also from 100ng per well) as the positive control were applied across an immunassay plate. An ELISA was performed to show which variants showed binding of a comparable capacity to the α340Ch antibody.

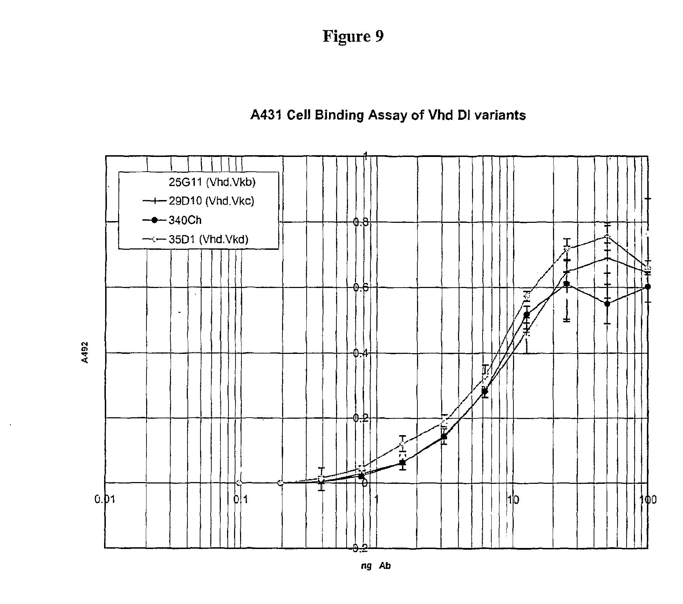

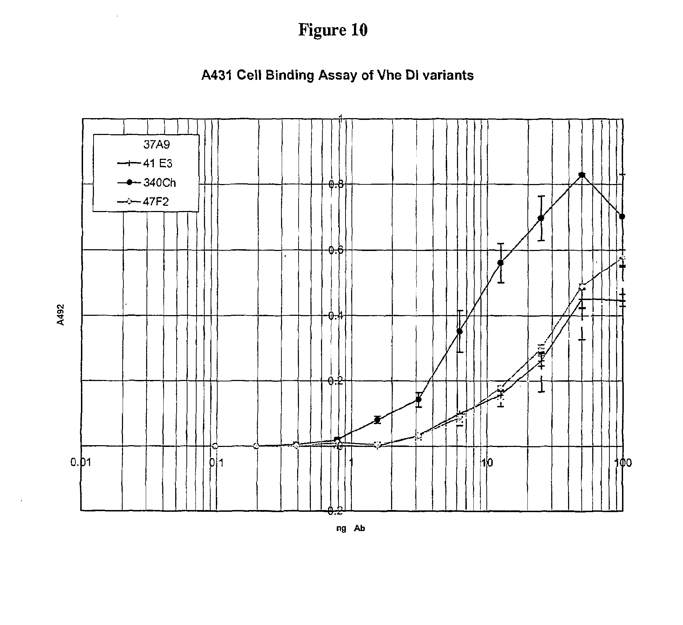

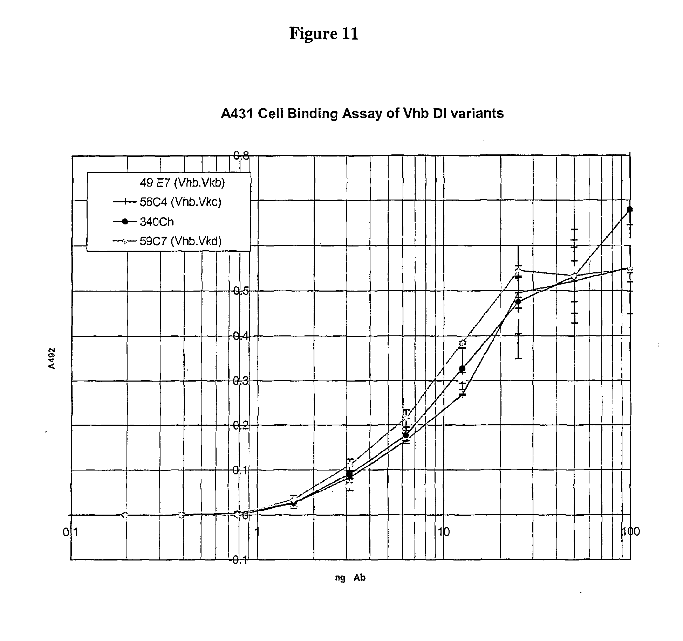

The results of the binding assays for the 3 Delmmunised α340 heavy chains combined with the 3 Delmmunised α340 light chains are shown in Figures 9 to 11. The antibody composed of DeImmunised heavy chain versions b and d combined with DeImmunised light chain version b, c and d showed equivalent binding to A431 compared to the chimaeric antibody, indicating that the mutations introduced to the V_{K} region do not compromise the EGFR binding of α340. However, the introduction of the mutation unique to V_{H}e (V-A in CDR3 of V_{H}) resulted in a loss of binding activity towards EGFR of around 3 to 4-fold.

The deimmunised version V_{H}d.V_{K}b was selected as lead deimmunised α340 antibody as it contained only one potential MHC epitope. Removal of this last epitope led to a considerable loss of EGFR binding by the antibody.

### Example 6 -Amino acid Homology Between SC100 Monoclonal Antibody and EGF

The Gene Jockey II software package for Macintosh by Biosoft was used for pairwise sequence comparison of protein sequences to identify areas of similarity/homology. When the CDRH3 region of SC100 was compared to the protein sequence of EGF, amino acid homology was observed in two distinct regions of EGF. However, when these regions were highlighted on the NMR resolved structure of EGF, they were found to be brought together by secondary structure and resemble SC 100 CDRH3.

### Example 7 - Demonstration of Inhibition of In Vitro Tumour Growth

A431 cells were maintained in RPMI 1640 supplemented with 10% foetal calf serum at 37°C and 5% Carbon dioxide. Confluent cultures of viable cells were harvested with trypsin/EDTA, washed and re-suspended at 5 x 10⁴ cells/ml.

100 µl aliquots of cell suspension were then dispensed into flat-bottomed 96-well plates together with increasing amounts of 340 mouse antibody, deimmunised SC100 (V_{H}dV_{K}d or V_{H}d,V_{K}b) antibody. These cultures were left for 5 days and the number of remaining viable cells determined by crystal violet staining. The results are the mean +SE for quadruplicate wells. Where error bars are not seen, this is because they are so small they are covered by the data point.

Both the V_{H}dV_{K}b and the V_{H}dV_{K}d SC100 clones were able to inhibit the growth of A431 cells more effectively than the mouse monoclonal antibody 340. Indeed, the V_{H}dV_{K}b clone showed 70% inhibition of cell growth.

## Claims

1. A humanised form of the mouse antibody 340, which mouse antibody is obtainable from the cell line deposited with the ECACC under accession number 97021428, the humanised form comprising the variable region of (i) V_{H}d as shown in Figure 6 and V_{K}d as shown in Figure 7, or (ii) V_{H}d as shown in Figure 6 and V_{K}b as shown in Figure 7.

2. An antibody as claimed in claim 1, comprising (i) V_{H}d and V_{K}d or (ii) V_{H}d and V_{K}b provided in a human antibody framework.

3. An antibody as claimed in claim 2, wherein the human antibody framework is the constant region of a human antibody.

4. A derivative of an antibody as claimed in any one of claims 1 to 3, which has 10 or fewer amino acid substitutions and is capable of binding to EGFR and inhibiting the growth of cells at a rate greater than antibody 340.

5. An antibody as claimed in any preceding claim linked to a coupling partner or effector molecule.

6. Nucleic acid encoding an antibody as claimed in any preceding claim.

7. A vector having one or more control sequences operably linked to a nucleic acid as claimed in claim 6 to control its expression.

8. A cell containing nucleic acid as claimed in claim 6 or a vector as claimed in claim 7.

9. A method of making an antibody, the method including expression from nucleic acid as claimed in claim 6.

10. A method as claimed in claim 9, comprising growing a host cell as claimed in claim 8 in culture under appropriate conditions which cause or allow expression of the antibody.

11. A pharmaceutical composition comprising an antibody as claimed in any one of claims 1 to 5 and a pharmaceutically acceptable carrier.

12. An antibody as claimed in any one of claims 1 to 5, or a nucleic acid as claimed in claim 6, for use in medicine.

13. The use of an antibody as claimed in any one of claims 1 to 5, or of a nucleic acid as claimed in claim 6, in the manufacture of a medicament for the treatment or prophylaxis of cancer.

## Patentansprüche

1. Humanisierte Form des Maus-Antikörpers 340, dessen Maus-Antikörper aus der Zelllinie, die bei der ECACC unter der Zugriffsnummer 97021428 hinterlegt ist, beschaffbar ist, wobei die humanisierte Form die variable Region von (i) V_{H}d, wie in Fig. 6 dargestellt, und V_{K}d, wie in Fig. 7 dargestellt, oder (ii) V_{H}d, wie in Fig. 6 dargestellt, und V_{K}b, wie in Fig. 7 dargestellt, enthält.

2. Antikörper nach Anspruch 1, welcher (i) V_{H}d und V_{K}d oder (ii) V_{H}d und V_{H}b aufweist, die in einer humanen Antikörperstruktur vorgesehen sind.

3. Antikörper nach Anspruch 2, wobei die humane Antikörperstruktur die konstante Region eines menschlichen Antikörpers ist.

4. Derivat eines Antikörpers nach einem der Ansprüche 1-3, das 10 oder weniger Aminosäuresubstitutionen aufweist und in der Lage ist, an EGFR zu binden und das Wachstum von Zellen stärker als der Antikörper 340 zu hemmen.

5. Antikörper nach einem der vorigen Ansprüche, der an einen Bindungspartner oder an ein Effektormolekül gebunden ist.

6. Nukleinsäure, die einen Antikörper nach einem der vorigen Ansprüche kodiert.

7. Vektor mit einer oder mehreren Steuersequenzen, der operabel an eine Nukleinsäure nach Anspruch 6 gekoppelt ist, um deren Expression zu steuern.

8. Zelle, die Nukleinsäure nach Anspruch 6 oder einen Vektor nach Anspruch 7 enthält.

9. Verfahren zur Herstellung eines Antikörpers, das die Expression von Nukleinsäure nach Anspruch 6 aufweist.

10. Verfahren nach Anspruch 9, welches die Züchtung einer Wirtszelle nach Anspruch 8 in Kultur unter geeigneten Bedingungen aufweist, die die Expression des Antikörpers bewirken oder ermöglichen.

11. Pharmazeutische Zusammensetzung, die einen Antikörpers nach einem der Ansprüche 1 bis 5 und einen pharmazeutisch akzeptablen Trägerstoff enthält.

12. Antikörper nach einem der Ansprüche 1 bis 5 oder eine Nukleinsäure nach Anspruch 6 zur medizinischen Nutzung.

13. Verwendung eines Antikörpers nach einem der Ansprüche 1 bis 5 oder einer Nukleinsäure nach Anspruch 6 bei der Herstellung eines Arzneimittels zur Behandlung oder Prophylaxe von Krebs.

## Revendications

1. Forme humanisée de l'anticorps murin 340, lequel anticorps murin peut être obtenu à partir de la lignée cellulaire déposée à l'ECACC sous le numéro d'enregistrement 97 021 428, la forme humanisée comprenant la région variable (i) de la V_{H}d comme présenté à la Fig. 6 et de la V_{K}d comme présenté à la Fig. 7, ou (ii) de la V_{H}d comme présenté à la Fig. 6 et de la V_{K}b comme présenté à la Fig. 7.

2. Anticorps suivant la revendication 1, comprenant (i) la V_{H}d et la V_{K}d ou (ii) la V_{H}d et la V_{K}b procurées dans une structure d'anticorps humain.

3. Anticorps suivant la revendication 2, dans lequel le squelette d'anticorps humain est la région constante d'un anticorps humain.

4. Dérivé d'un anticorps suivant l'une quelconque des revendications 1 à 3, qui présente 10 ou moins de substitutions d'acides aminés et peut se lier à l'EGFR et inhiber la croissance de cellules à un niveau supérieur à celui de l'anticorps 340.

5. Anticorps suivant l'une quelconque des revendications précédentes, lié à un partenaire de couplage ou à une molécule effectrice.

6. Acide nucléique codant pour un anticorps suivant l'une quelconque des revendications précédentes.

7. Vecteur présentant une ou plusieurs séquences de contrôle liées de manière fonctionnelle à un acide nucléique suivant la revendication 6 pour un contrôle de son expression.

8. Cellule contenant un acide nucléique suivant la revendication 6 ou un vecteur suivant la revendication 7.

9. Procédé de fabrication d'un anticorps, le procédé comprenant une expression à partir d'un acide nucléique suivant la revendication 6.

10. Procédé suivant la revendication 9, comprenant la croissance d'une cellule hôte suivant la revendication 8 en culture dans des conditions appropriées qui provoquent ou permettent l'expression de l'anticorps.

11. Composition pharmaceutique comprenant un anticorps suivant l'une quelconque des revendications 1 à 5 et un vecteur pharmaceutiquement acceptable.

12. Anticorps suivant l'une quelconque des revendications 1 à 5, ou un acide nucléique suivant la revendication 6, pour une utilisation en médecine.

13. Utilisation d'un anticorps suivant l'une quelconque des revendications 1 à 5, ou d'un acide nucléique suivant la revendication 6, dans la fabrication d'un médicament pour le traitement ou la prophylaxie d'un cancer.
